# EUROPEAN PATENT APPLICATION

(11) **EP 3 674 402 A1**
(43) Date of publication of application: **01.07.2020**
(21) Application number: 18382998.5
(22) Date of filing: 27.12.2018
(51) Int. Cl.: C12N 9/10, C12N 1/20, C12P 19/04, C12R 1/01

(54) **WEISSELLA VIRIDESCENS STRAIN AND USES THEREOF FOR THE SYNTHESIS OF EXOPOLYSACCHARIDES**

(71) Applicant: Sant Dalmai, S.A.U., 17183 Sant Dalmai Gerona (ES)
(72) Inventor: Panella Gubau, Josep, 17458 Fornells de la Selva (Girona) (ES); Segarra Manzano, Silvia, 46011 Valencia (ES); Del Cerro Fernández, Carolina, 46006 Valencia (ES); Álvarez Pérez, Beatriz, 46001 Valencia (ES); Navarro Barrera, Verónica, 46370 Chiva (Valencia) (ES); Tortajada Serra, Marta, 46015 Valencia (ES); Ramón Vidal, Daniel, 46183 La Eliana (Valencia) (ES)
(74) Representative: Pons

(57) **Abstract**

The present invention relates to a strain of the species *Weissella viridescens* with accession number CECT 9735 that displays a significantly increased ability to synthesize exopolysaccharides (EPS) compared to other strains of the same genus in the prior art. Specifically, the strain of the invention described herein comprises a polynucleotide sequence encoding an enzyme responsible for the synthesis of said EPS, wherein said sequence is comprised in a mobile element and not in the cell genome. Furthermore, this invention relates to the EPS, *per se,* secreted by said cell, as well as the said EPS production method.

## Description

The present invention relates to a strain of the species *Weissella viridescens* with accession number CECT 9735 that has the ability to produce exopolysaccharides (EPS) from sucrose. Therefore, the present invention could be framed within the field of industrial biotechnology, in particular the production of biopolymers.

### STATE OF THE ART

Exopolysaccharides (EPS) are polymers of monosaccharides produced by bacteria able to demonstrate strong or weak adherence to the cell surface or are released into the extracellular medium forming a matrix. Depending on their chemical composition, EPS can be classified as homopolysaccharides, consisting of units of a single type of monosaccharide, or heteropolysaccharides, consisting of units of several monosaccharides. These monosaccharides are mainly glucose, fructose, galactose, ramnose or arabinose and can be linked by different types of bonds (α or β), both in the main chain and in the branches. Depending on the composition of monosaccharides, the type of bond and the carbon position of the main bond, there is a wide variety of polymers with different names and produced by different bacteria. It is interesting to note that new structures of these compounds are continually being described.

Currently, there is interest in investigating and characterizing EPS due to their positive or negative influence on foods containing these bacteria, in addition to the potential health benefits attributed to them.

Numerous EPS-producing lactic-acid bacteria belonging to different genera have been identified (Zafar S. B., et al. Journal of Genetic Engineering and Biotechnology. 2018; (16): 17-22). These include those of the genus *Weissella,* which are capable of producing some EPS or not, depending on the specific species (Fusco V., etal. Frontiers in Microbiology. 2015; (6): 155).

Knowledge and characterization of bacteria, as well as the EPS they produce, helps the development of industrial processes to produce these compounds. For example, patent WO2015117624A1 describes a method of producing an EPS of interest, dextran, using the bacterium *Weissella cibaria.*

Therefore, the state of the art evidences the need to provide useful microorganisms for the industrial production of EPS of interest with higher yields than those described in the prior art, for their appropriate and efficient industrial use.

### DESCRIPTION OF THE INVENTION

The inventors of the present invention have identified a strain of the species *Weissella viridescens* isolated from meat products which, surprisingly, is capable of producing exopolysaccharides (EPS), with a higher yield than that known to date for bacteria of the same genus, using sucrose as carbon source. This capacity is bestowed on the said strain by the presence of a nucleotide sequence that encodes an enzyme capable of producing the said EPS, more specifically, the nucleotide sequence encoding that enzyme is found in a mobile element of DNA, more specifically, that nucleotide sequence is found in a plasmid, outside the genome of the strain. This feature allows the application of this strain to obtain EPS in processes with a superior yield to other microorganisms producing these compounds, as will be demonstrated below.

Therefore, in a first aspect, the present invention relates to a lactic-acid bacterial strain, referred to hereinafter as, strain of the invention, preferably of the genus *Weisella,* more preferably of the species *Weissella viridescens,* and even more preferably of the strain *W. viridescens* CECT 9735.

The strain of the invention was deposited on October 3, 2018 under the Budapest Treaty in the Spanish Type Culture Collection (CECT), as International Depositary Authority (Building 3 CUE, Parc Científic Universitat de Valencia, C/ Catedrático Agustín Escardino, 9, 46980, Paterna,Valencia, Spain). The accession number assigned is CECT 9735 (hereinafter "strain of the invention CECT 9735" or *"Weissella viridescens* CECT 9735").

*W. viridescens* CECT 9735 is a Gram-positive bacterium of spherical or lenticular irregular rod shape. The strain of the invention belongs to the Kingdom: Bacteria; Phyla: Firmicutes; Class: Bacilli; Order: Lactobacillales; Family: Leuconostocaceae; Genus: Weissella. The said strain was isolated from meat products.

As shown throughout this document, the strain of the invention is capable of producing EPS, with a higher yield than any known so far for bacteria of the same genus. This is due to the presence of a nucleotide sequence encoding an enzyme capable of producing said EPS, which is located in a mobile element, specifically in a plasmid, and not in its genome.

Thus, in another preferred embodiment, the strain of the invention comprises the polynucleotide of the invention comprising SEQ ID NO: 1. In another more preferred embodiment, the strain of the invention comprises the polynucleotide comprising SEQ ID NO: 1, preferably in a mobile element or plasmid, more preferably, where the said mobile element or plasmid is extrachromosomal. In another more preferred embodiment, the mobile element or extracromosomal plasmid comprising the polynucleotide of the invention is defined in SEQ ID NO: 3.

Thus, in another aspect, the invention relates to a sequence of nucleotides, from now on, nucleotide sequence of the invention or polynucleotide of the invention, which encodes for an enzyme capable of producing EPS and comprising SEQ ID NO: 1.

In another particular embodiment, the nucleotide sequence of the polynucleotide of the invention consists of the nucleotide sequence shown in SEQ ID NO: 1.

In another particular embodiment, the polynucleotide of the invention is a substantially homologous and functionally equivalent variant of the polynucleotide whose nucleotide sequence is shown in SEQ ID NO: 1. In this respect, the polynucleotide of the invention corresponds to: a) nucleic acid molecules of the isolated polynucleotide sequence consisting of SEQ ID NO: 1, or its complementary chains, b) nucleic acid molecules whose complementary chain is capable of hybridizing under astringent conditions with a polynucleotide sequence of (a), c) nucleic acid molecules whose sequence differs from a) and/or (b) due to the degeneration of the genetic code.

As used in the description, a polynucleotide is "substantially homologous" to the polynucleotide of SEQ ID NO: 1 when its nucleotide sequence has a degree of identity with the nucleotide sequence shown in SEQ ID NO: 1 of at least 90%, more preferably of, at least, 91 %, 92%, 93%, 95%, 96%, 97% or 98%, and still more preferably of at least 99%. The degree of identity between two nucleotide sequences can be determined by conventional methods, for example, by using standard algorithms of sequence alignment known in the prior art, such as, for example, BLAST (Altschul S. F. *et al.* 1990). By way of example, a polynucleotide that is substantially homologous to the polynucleotide whose nucleotide sequence comprises, or consists of, the nucleotide sequence shown in SEQ ID NO: 1 can be constructed on the basis of the nucleotide sequence shown in said SEQ ID NO: 1 by introducing, for example, nucleotides or codons encoding amino acids constituting substitutions, which are conservative or non-conservative with respect to the amino acids encoded by the nucleotide sequence shown in SEQ ID NO: 1. Other illustrative examples of possible modifications include the insertion of one or more nucleotides into the sequence, the addition of one or more nucleotides at either end of the sequence, or the deletion of one or more nucleotides at either end or inside the sequence. In the definition used in this description, a polynucleotide is "functionally equivalent" to the polynucleotide whose nucleotide sequence comprises, or consists of, the nucleotide sequence shown in SEQ ID NO: 1 when encoding a protein capable of producing EPS.

In a particular embodiment, the polynucleotide of the invention is a substantially homologous and functionally equivalent variant of the invention, that is, it is a polynucleotide whose nucleotide sequence has a degree of identity of at least 90%, more preferably of at least 91%, 92%, 93%, 94%, 95%, 96%, 97% or 98%, and even more preferably at least 99%, with respect to the nucleotide sequence shown in said SEQ ID NO: 1 and that encodes a peptide capable of producing and secreting EPS.

More preferably, the polynucleotide of the invention comprises SEQ ID NO: 1, more preferably, the polynucleotide of the invention consists of SEQ ID NO: 1.

The polynucleotide of the invention can be found either as such or as part of a gene construction, henceforth gene construction of the invention, which comprises said polynucleotide of the invention. The genetic construction of the invention can incorporate, operatively joined, a regulatory sequence of the expression of the polynucleotide of the invention, thus constituting an expression cassette. As used in this description, the expression "operatively joined" means that the peptide of the invention, encoded by the polynucleotide of the invention, is expressed in the correct reading frame controlled by the expression control sequences or regulators. The control sequences are sequences that control and regulate the transcription and, when applicable, the translation of the peptide of the invention, and include promoter sequences, coding sequences for transcriptional regulators, ribosome binding sequences (RBS) and/or transcription terminator sequences. In one particular embodiment, said expression control sequence is functional in prokaryote cells and organisms, for example, bacteria, etc., while, in another particular embodiment, such an expression control sequence is functional in cells and eukaryotic organisms, for example, insect cells, plant cells, mammalian cells, etc. Advantageously, the construction of the invention further comprises a marker or gene encoding a motif or a phenotype which permits the selection of the host cell transformed with such construction. The genetic construction of the invention can be obtained by the use of techniques widely known in the state of the art (Sambrook *et al.,* 2001).

The expression "gene construction " or "nucleic acid construction", as used herein, refers to a functional unit necessary for the transfer or the expression of a nucleotide sequence or gene of interest, in the present document, the nucleic acid sequence of the invention as described above, and regulatory sequences, including, for example, a promoter, operationally bound to the sequence that encodes the protein. Therefore, for the purposes of this invention, gene construction relates to a bicatenarian nucleic acid molecule, which is isolated from a natural nucleic acid or which is artificially modified to contain segments of nucleic acids. The expression "nucleic acid construction" is synonymous with the expression "expression cassette" when the nucleic acid construction contains the control sequences required for the expression of the coding sequence.

The genetic construction of the invention can be inserted into an appropriate vector. Therefore, in another aspect, the invention is related to a vector, such as a recombinant vector, henceforth vector of the invention, which comprises the polynucleotide of the invention or the gene construction of the invention. The choice of vector will depend on the host cell in which it to be inserted later. In a particular embodiment, the vector of invention is an expression vector. The term "expression vector", also known as "expression construction" or "plasmid", refers to a linear or circular DNA molecule comprising the nucleic acid sequence of the invention and which is operatively linked to additional segments allowing the transcription of the encoded peptide. A plasmid is usually used to insert a specific gene into a target cell. Once the expression vector is inserted in the cell, the protein that is encoded by the gene is produced by the cell transcription and translation machinery. The vector is often genetically engineered to contain regulatory sequences that act as enhancer and promoter regions and lead to an efficient transcription of the gene carried in the expression vector. The purpose of an expression vector or plasmid is to produce large quantities of stable messenger RNA and therefore protein. Expression vectors are basic tools of biotechnology and protein production, such as enzymes. The expression vector of the invention is inserted in a host cell so that the vector is retained as an integral part of the chromosome or as an extra-chromosomal self-replicating vector. In a preferred embodiment of the invention, the expression vector or plasmid is retained as an extra-chromosomal self-replicating vector or plasmid. Illustrative, non-limiting examples of vectors in which the polynucleotide of the invention or the gene construction of the invention can be inserted are plasmids, phages, cosmids, phagemids, yeast artificial chromosomes (YAC), bacterial artificial chromosomes (BAC), human artificial chromosomes (HAC) or viral vectors, such as adenovirus, retrovirus or lentivirus. The vector of the invention can be obtained by conventional methods known by technicians in the art (Sambrook *et al.*, 2001). In a particular embodiment, said vector is a useful vector for transforming competent cells of *Escherichia coli.* The vector of the invention can be used to transform cells that can be transformed by that vector. Said cells may be prokaryotes or eukaryotes. The vector of the invention can be used to transform eukaryotic cells, such as yeast, for example, *Aspergillus niger, Pichia pastoris,* etc., or microalgae, for example, *Chlamydomonas reinhardtii,* etc., or prokaryotic cells, such as bacteria, for example, *E. coli, Pseudomonas putida, Lactobacillus sp., Bifidobacterium sp., Bacillus subtilis,* etc.

More preferably, the plasmid of the invention comprises the sequence SEQ ID NO: 1.

Thus, the gene construction of the invention that comprises the SEQ ID NO: 1 can be used to obtain transgenic cells capable of expressing the peptide encoded by said SEQ ID NO: 1.

In this respect, another aspect of the present invention is related to a transgenic host cell, henceforth the transgenic cell of the invention, which comprises the polynucleotide of the invention, the gene construction of the invention, the expression cassette of the invention, or the plasmid of the invention, and is able to express the protein of the invention (defined below). The term "cell", as used here, refers to the smallest unit that maintains the fundamental properties of life, and, comprises prokaryotic, eukaryotic cells, as well as cells of single-celled (e.g., bacteria) and multicellular (e.g., animals, plants) organisms. Thus, by way of illustration, the transgenic cell of the invention can be a eukaryotic cell, such as a plant tissue cell, a yeast, a microalgae, etc., a prokaryotic cell, such as a bacterium, for example, *E. coli,* etc. The transgenic cells of the invention can be obtained by conventional methods known by technicians in the art (Sambrook *et al.*, 2001). In a particular embodiment, this transgenic cell of the invention is a recombinant host cell, which has been transformed with the vector of invention.

For the purposes of the present invention, the terms "endogenous" or "homologous" refer both to genes and to proteins that are naturally found in a cell, that is to say, without any human intervention. In addition, these terms also refer to those same genes or proteins that, once isolated from the organism, can be reintroduced (transgene) through genetic engineering.

For the purposes of this invention, the term "heterologous" refers to a nucleic acid derived from a different species or, if derived from the same species, a nucleic acid that is substantially modified from its native form. For example, a promoter that is operatively linked to a heterologous structural gene belongs to a different species from which the structural gene was originally obtained, provided it originates from deliberate human intervention. If they belong to the same species, they must be substantially modified in their original form in one to several heterologous genes. A heterologous protein may originate from a different species or from the same species, provided it originates from deliberate human intervention.

As used herein, the term "recombinant" refers to a polynucleotide or polypeptide that is not naturally produced in a host cell. In certain embodiments, the "recombinant cells" or "transgenic cells" express genes that are not found in an identical form as the native or wild (i.e., non-recombinant) form of the cell and/or express native genes whose expression would otherwise be increased, decreased or knocked-out due to the deliberate human intervention. Recombinant cells contain at least one recombinant polynucleotide or polypeptide. A nucleic acid construction comprising the nucleic acid itself and the elements necessary for its expression, nucleic acid (e.g. a polynucleotide), cell or polypeptide are referred to here as "recombinant" when of non-natural, artificial or processed origin.

In the transgenic cell of the invention more than one copy of the nucleic acid sequence of the present invention can be inserted to increase the production of the gene product. The number of copies of the polynucleotide can be increased by integrating at least one additional copy of the sequence into the transgenic host cell or by including a selectable marker gene amplifiable with the polynucleotide, where cells containing amplified copies of the selectable marker gene and therefore additional copies of the polynucleotide can be selected by growing the cells in the presence of the appropriate selectable agent. The procedures used to bind the elements described above in order to construct the recombinant expression vectors referred to in this invention are well known by experts in the art.

In another preferred embodiment, the transgenic cells of the invention are characterized by the overexpression of the peptide encoded by the nucleotide sequence SEQ ID NO: 1, with the ability to secrete EPS. More preferably, the transgenic cells of the invention are characterized by an increase in the production of EPS compared to parental or wild-type cells that do not comprise the nucleotide sequence of the invention SEQ ID NO: 1, which encodes for the protein comprising the SEQ ID NO: 2 and which is capable of producing and secreting EPS.

For the purposes of this invention, the terms "increased expression" or "overexpression" may be used interchangeably throughout this document and refer to any form of expression that is additional to or greater than the original expression level in a parental or wild cell. For the purposes of the present invention, in increasing order of preference, overexpression is at least 10%, 20%, 30%, 40%, 50%, 60%, 70%, 80%, 85%, 90%, 95%, 96%, 97%, 98%, 99% or 100% compared to expression in the parental or wild cells. Methods to increase the expression of genes or gene products are well documented in the art and include, for example, overexpression driven by appropriate promoters, the use of transcription enhancers or translation enhancers. Nucleic acid isolates can also serve as promoters or enhancers, insertable in an appropriate position in a non-heterologous form of a polynucleotide in order to upregulate the expression of a nucleic acid that encodes the polypeptide of interest. For example, endogenous promoters can be altered *in vivo* by mutation, deletion and/or substitution; also isolated promoters can be inserted in a polynucleotide sequence that encodes for a gene of interest to control its expression.

For the purposes of this invention, the scope of these terms is intended to cover the increase in the expression of both homologous and heterologous enzymes.

In some embodiments, the increase in expression includes a high transcription rate and/or a high gene level compared to the homologous transcription rate of that gene. In some other embodiments, a heterologous gene is introduced into a host cell to induce increased expression of a gene encoding a homologous enzyme. In certain embodiments, the heterologous gene is a gene that has been modified to increase the expression of the gene product. In some embodiments, the term also covers polypeptide secretion from a cell.

An expert in the art knows the different tools and routine techniques for overexpression or increased expression of a gene and/or protein.

The "host cell" or "transgenic cell", as used herein, includes any cell type that is susceptible to transformation, transfection, transduction and similar to the genetic construction of the invention. The host cell can be eukaryotic, such as a mammal, insect, plant, bacterial, or fungal cell. In a preferred embodiment, the host cell is a bacterial cell. In a more preferred embodiment, the bacterial host cell is selected from the list consisting of: *Bacillus subtilis, Escherichia coli, Pseudomonas pútida, Lactobacillus sp and Bifidobacterium sp,* among others.

In another aspect, the invention is related to a peptide, hereinafter "peptide of the invention", whose amino acid sequence corresponds to the amino acid sequence encoded by the polynucleotide of the invention.

The term "peptide", as used herein, refers to an amino acid chain bound by peptide bonds, regardless of their length, and includes both amino acid chains of 10 or less amino acids (in some publications identified as "oligopeptides") and amino acid chains of more than 10 amino acids, for example, over 100 amino acids.

In a particular embodiment, the peptide of the invention comprises the sequence of amino acids shown in the SEQ ID NO: 2. The amino acid sequence shown in the SEQ ID NO: 2 corresponds to the sequence encoded by the polynucleotide whose nucleotide sequence is shown in the SEQ ID NO: 1. Said peptide is capable of secreting EPS. In another preferred embodiment, the peptide of invention consists of the SEQ ID NO: 2.

According to the meaning used in this description, a peptide is "substantially homologous" to the peptide comprising the SEQ ID NO: 2, when its amino acid sequence has a degree of identity with respect to the amino acid sequence shown in the SEQ ID NO: 2 of at least 90%, more preferably of at least 91%, 92%, 93%, 95%, 96%, 97% or 98%, and even more preferably of at least 99%. The degree of identity between two amino acid sequences can be determined by conventional methods, for example, by using standard algorithms of sequence alignment, known in the prior art, such as, for example, BLAST (Altschul S. F. *et al.* 1990). By way of illustration, a peptide that is substantially homologous to the peptide whose nucleotide sequence comprises, or consists of, the amino acid sequence shown in the SEQ ID NO: 2 can be constructed on the basis of the amino acid sequence shown in the SEQ ID NO: 2 by inserting, for example, amino acids that constitute conservative or non-conservative substitutions with respect to the amino acids present in the amino acid sequence shown in the SEQ ID NO: 2. Other illustrative examples of possible modifications include the addition of one or more amino acids at either end (amino or carboxyl), the insertion of one or more amino acids in the sequence, or the deletion of one or more nucleotides at either end or inside the sequence. In the meaning used in this description, a peptide is "functionally equivalent" to the peptide whose amino acid sequence comprises, or consists of, the amino acid sequence shown in the SEQ ID NO: 2 when it is able to synthesize and secrete EPS. In a particular embodiment of the peptide comprising the SEQ ID NO: 2 of the invention, it is characterized by a high sequence identity, preferably more than 95% sequence identity, with peptides known in the prior art and where these state-of-the-art peptides have dextransacarase activity. Therefore, the peptide comprising the SEQ ID NO: 2 of the invention may also present such dextransacarase activity. The dextransacarase activity of the peptide of the invention encoded by the polynucleotide that is functionally equivalent to the polynucleotide whose nucleotide sequence comprises, or consists of, the nucleotide sequence shown in SEQ ID NO: 1, can be determined by conventional determination methods of said activity, such as, for example, through the analysis of the concentration of reducing sugars using the 3,5 dinitrosalicylic acid (DNS) method (Miller, G. L. Analytical Chemistry. 1959; 31: 426-428).

The term 'dextransacarase' refers to a peptide or enzyme in the glucosyltransferase Group (E. C. 2.4.1.5) capable of catalysing the synthesis of an exopolysaccharide using sucrose (α-D-glucopyranosyl-(1-2)-β-D-fructofuranoside) as a substrate.

In another particular embodiment, the peptide of the invention is a substantially homologous and functionally equivalent variant of the peptide whose amino-acid sequence is shown in the SEQ ID NO: 2.

The person skilled in the art will understand that the amino acid sequences referred to in this description can be chemically modified, for example, by means of chemical modifications which are physiologically relevant, such as, phosphorylations, acetylations, etc. Also, the person skilled in the art will understand that there are peptide fragments satisfying such conditions within these variants which are substantially homologous and functionally equivalent to the peptide whose amino acid sequence is displayed in the SEQ ID NO: 2.

The peptide of the invention can be obtained by conventional methods known by the technicians in the art. Although the said peptide of the invention can be isolated from the strain of the invention, particularly from strain *Weissella viridescens,* preferably *W. viridescens* strain CECT 9735, in another particular embodiment, said peptide of the invention can also be obtained by methods based on recombinant DNA technology.

In another aspect, the invention is related to a procedure to obtain a peptide of the invention, which includes cultivating the CECT 9735 strain of the invention, or the transgenic cell of the invention, under conditions that allow the production of said peptide and, if desired, recover said peptide from the culture medium. The conditions for optimising the culture of these cells will depend on the cell used. The expert in the art knows such conditions. The procedure for producing the peptide of the invention includes, optionally, the isolation and purification of said peptide of the invention.

In another aspect, the present invention relates to the use of the CECT 9735 strain, or the transgenic host cell of the invention, as described above, for the procurement of EPS.

The term "exopolysaccharide" or "EPS " means a polymer composed of more than 10 monosaccharides secreted by an organism. These units may be glucose, mannose, ribose, arabinose, etc. linked by o-glucosidic bonds in both the main chain and the branched chains. EPS are classified mainly according to their composition in heteropolysaccharides (if they consist of more than one type of monosaccharide) and homopolysaccharides (consisting of a single type of monosaccharide). The properties of these EPS are determined by: (i) the type of bond and monosaccharide units forming it; (ii) its degree and type of branching; and (iii) its molecular mass and conformation.

In another aspect, the present invention relates to a bacterial EPS isolated from a *Weissella viridescens* CECT 9735 culture.

As shown in Example 4, CECT 9735 strain of the invention produces a significant increase in EPS production of almost 50% compared to other strains of the same genus and/or species, such as *W. cibaria* CECT 7032, *W. cibaria* CECT 4710 or *W*. *viridescens* CECT 283.

In order to obtain said EPS production yields, the CECT 9735 strain of the invention is cultured under appropriate conditions. Thus, in another aspect, the present invention relates to a method of obtaining EPS characterized by the following steps:
a) cultivation of the strain of the invention or of the transgenic host cell of the invention, in a culture medium that allows its growth; and
b) extraction by precipitation procedure of the EPS synthesised by the cell of step a).

The term "culture medium" means a solid and/or liquid food containing the nutrients required for bacterial growth.

In order to obtain EPS by growing the strain of the invention, or the transgenic host cell of the invention, the culture medium must contain sources of carbon, nitrogen and inorganic salts, using the procedures known in the art. The culture medium may contain other additional components commonly used in the prior art and considered necessary for the growth of the strain or of the transgenic cell of the invention and the EPS synthesis obtained by the cultured of the cells mentioned above.

In a preferred embodiment of the method for obtaining EPS by growing the strain CECT 9735, this is characterized in that the stage a) is carried out at a pH of between 6.5 and 7.5, preferably at 7.2; and at a temperature of between 28 ° C and 35 ° C, preferably at 30 °C.

In another particular embodiment, the CECT 9735 strain of the invention is cultured under anaerobic conditions.

The strain of the invention or the transgenic host cell of the invention can be cultivated in a suitable, solid or liquid nutrient medium to produce the enzymes of the invention, using well-known procedures in the art. For example, the cell can be grown on a small scale or on a large scale (including continuous, discontinuous or *batch* fermentation, discontinuous or *fed-batch* or solid state culture) carried out in a laboratory or industrial bioreactor, in an appropriate medium and under conditions that enable the enzyme of the invention to be expressed and/or isolated. If the enzyme is secreted, together with other enzymes or proteins in the nutrient medium, they can be recovered directly from the medium.

With regard to the techniques for isolating the synthesized EPS, the precipitation of the EPS with cold ethanol is highlighted, although any technique known by an expert in the art is applicable whereby the EPS of interest can be separated from the culture medium, correctly maintaining its physical-chemical properties.

In another aspect, the present invention relates to an EPS characterised by glucose, mannose and arabinose, in a molar ratio of approximately 3.1: 1: 0.1.

In another preferred embodiment, the EPS has an elemental composition, in percentages by weight, of approximately at least 30-45% C; 1-10%H; 1-15% N, and 0.05-2% S. More preferably, the EPS of the invention has an elemental composition, in percentages by weight, of at least 37.7% C, 3.1% H, 5.25% N and 0.08% S. In another preferred embodiment, the EPS comprises an average molecular weight *Mw* of between 100 and 150 kDa, an average molecular mass number (Mn) of between 50 and 100 kDa and a polydispersity index of between 1.5 and 2.

In another particular embodiment, the EPS has a Fourier-transformed infrared spectrum (in cm⁻¹) where the maximum absorbance was observed at a peak at 1013.8 cm⁻¹, followed by a wide peak at 3298 cm⁻¹. Intense bands were also observed at approximately 2910, 2069, 1648.7, 1456, 1412, 1351, 1237 and 912 cm⁻¹.

In another preferred embodiment, the EPS includes the following bonds:

| **Types of bonds** | **Area (%)** |
|---|---|
| Hex*p*-(1 → | 7.1 |
| →2)-Man*p*-(1→ | 1.3 |
| →3)-Man*p*-(1→ | 0.5 |
| →6)-Hex*p*-(1→ | 81.7 |
| →4.6)-Hex*p*-(1→ | 0.5 |
| →3.6)-Man*p*-(1→ | 9.1 |

In a particular embodiment, EPS comprises at least 70% glucose, 20% mannose, and at least 3% arabinose.

Another aspect of the invention relates to compositions comprising the EPS of the invention, hereinafter, compositions of the invention.

EPS are widely used in the chemical, food and pharmaceutical industry for their emulsifying and functional properties, biocompatibility and biodegradability. EPS are part of many products such as herbicides, functional foods, nutraceuticals, cosmeceuticals, pharmaceutical active substances (such as immunomodulators and anticoagulants) or insecticides, among others.

In one particular embodiment, the EPS of the invention is used in the food industry, preferably as a thickening agent, stabilizing agent, emulsifying agent, gelling agent, such as hydrocolloids, such as inclusion bodies (for example, for the controlled release of vitamins, aromatic compounds, etc.).

In another particular embodiment, the EPS of the invention is used in the pharmaceutical and biomedical industry and environmental engineering, such as for example bio-adhesive, thickening agent, stabilizing agent, emulsifying agent, agent, gelling agent, binder (for example, in toothpaste, lipstick, creams, implants, etc). In particular, the benefits described in the field of medicine include the use of EPS composed of mannose and glucose in the form of highly soluble fibres. Said fibres have an exceptional capacity to capture water, forming highly viscous solutions. Glucomannans are efficient satiating agents, intestinal transit regulators (improving constipation by increasing faecal volume), hypocholesterolemic agents (interfering with the transport of cholesterol and bile acids) and regulators of insulin metabolism. In a preferred embodiment, EPS is used in the food industry, preferably as an excipient and/or additive.

In another preferred embodiment, the composition of the invention may also include other EPS and/or active substance, as well as excipients and / or additives.

Thus, the compositions of the invention can be compositions for use in personal care, pharmaceutical compositions, domestic products, industrial products or foodstuffs.

Throughout the description and claims, the word "comprise" and its variants are not intended to exclude other technical characteristics, additives, components or steps. For experts in the art, other objects, advantages and characteristics of the invention shall be made known partially from the description and partially from the practice of the invention. The following examples and figures are provided as way of example, and are no way intended to limit this invention.

### DESCRIPTION OF THE FIGURES

**Fig. 1****.** Photographs of serial cultivation of the CECT 9735 strain under anaerobic conditions at 30°C in medium VWA sucrose at pH 7.2 and pH 5. Detail of serial cultivation 4 (**A**) and serial cultivation 5 (**B**) of the CECT 9735 strain cultivated at pH 5.

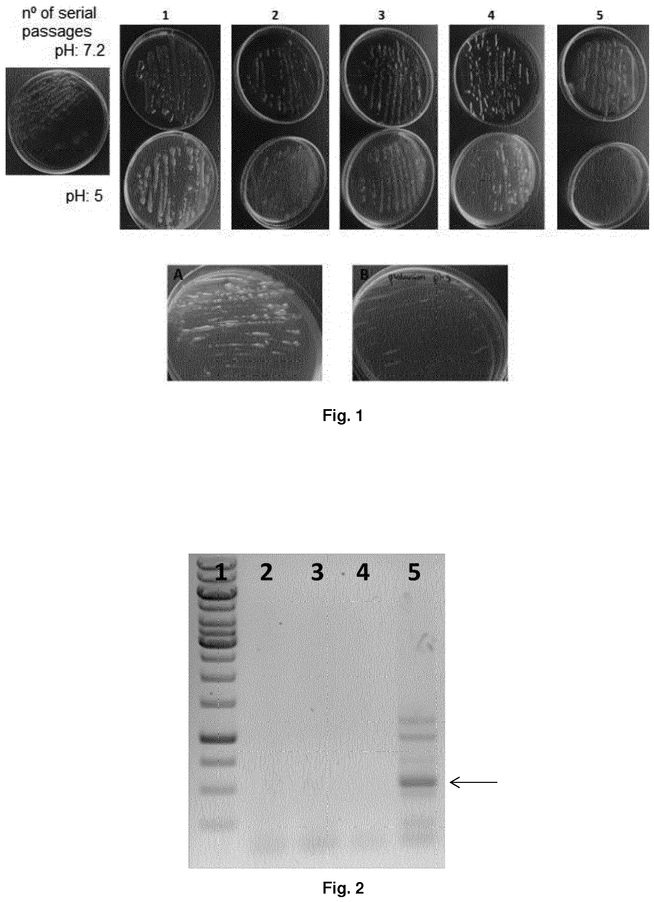

**Fig. 2****.** Photograph of a gel obtained by electrophoresis. **1.** molecular weight marker 1Kb; **2.** genomic DNA of the CECT 9735 strain of the invention grown under conditions with loss of EPS production capacity (pH 5, 30°C, anaerobiosis); **3.** Plasmid DNA of the CECT 9735 strain of invention grown under conditions with loss of EPS production capacity (pH 5, 30°C, anaerobiosis); **4.** Genomic DNA of the CECT 9735 strain of the invention grown under conditions with EPS production capacity (pH 7.2, 30°C, anaerobiosis); **5.** Plasma DNA of the CECT 9735 strain of the invention grown under conditions with EPS production capacity (pH 7.2, 30°C, anaerobiosis); **6.** Negative control, mixture of PCR components, but no DNA.
**Fig. 3****.** EPS production (g/L) by culturing the strains *W. viridescens* CECT 9735, *W*. *cibaria* CECT 7032, *W. cibaria* CECT4710, *W. viridescens* CECT 283 and *W*. *viridescens* P28B.8.

### EXAMPLES

Below the invention will be illustrated by tests performed by the inventors, which show the advantages of the products of the invention.

### Example 1. Identification and characterisation of EPS-producing bacterial strain CECT 9735.

From a meat product isolate, said isolates were grown on MRS culture medium and genomic DNA was isolated followed by direct amplification by PCR of the 16S rRNA gene. Subsequently, the 16S rRNA gene was partially sequenced, with readings in both directions, and the sequences were analysed.

Sequence analysis was performed using BLAST. The result obtained from collecting the extension of the overlapping fragment, the similarity percentage and the sequence accession number of the species type strain with the highest degree of identity, revealed that the bacterium belongs to the genus *Weisella* and the species *Weissella viridescens* with a similarity of 1014/1014 pb (100%) on the sequence AB023236 (NRIC type strain 1536) and, specifically, *W. viridescens* CECT 9735.

### Example 2. Identification and characterisation of the gene encoding the enzyme capable of producing EPS in strain CECT 9735.

Genomic DNA was isolated from the CECT 9735 strain of the invention and the genome of said strain was subjected to complete sequencing. Once the DNA was purified and concentrated, the DNA being clean of inhibitors, metals and pigments, the sequencing libraries were prepared. For this step the DNA was fragmented and selected according to its size. The samples were sequenced using the PacBio platform, using P6-C4 polymerase and a 4-hour data acquisition time. Once the quality sequences were obtained, the Pacific Bioscience recommendations were followed with the aim of performing a *de novo* assembly. Blast sequence analysis of the results obtained demonstrated that the bacterium possesses a homologous sequence with genes encoding dextransacarase and that it is located in a plasmid or extracromosomal mobile element, not forming part of the cell genome.

The complete sequence of the plasmid comprising the gene encoding the enzyme responsible for the EPS synthesis corresponds to SEQ ID NO: 3.

In addition to the nucleotide sequence SEQ ID NO: 1 encoding the enzyme responsible for EPS synthesis comprising SEQ ID NO: 2, the inventors have identified another 7 nucleotide sequences encoding 7 other genes in this plasmid.

The nucleotide sequence of the invention encoding the peptide responsible for EPS synthesis represents in strain *W. viridescens* CECT 9735 of the invention 25% of the complete sequence of the plasmid (SEQ ID NO: 3), with 3042 bp of the gene coinciding with the base pairs of the plasmid from 3042 bp to 1 bp.

Comparing the nucleotide sequence (SEQ ID NO: 1) that encodes the enzyme responsible for EPS synthesis (SEQ ID NO: 2) of the strain of the invention, compared to the sequences recorded in the NCBI database by Blast analysis, it is observed that the sequence is preserved in different strains of *W. cibaria* and in a strain of *Lactobacillus fermentum* with a homology of between 96% and 99%, but no homology has been found with any strain of *W. viridescens* **(Table 1**).

**Table 1. Homology of the dextransacarase gene in other species.**

| **Organism** | **Strain** | **Max. Score** | **Score total** | **Coverage (Query cover)** | **E-value** | **Identity** | **GenBank** |
|---|---|---|---|---|---|---|---|
| *Weissela cibaria* | LBAE-K39 | 5607 | 5607 | 100% | 0.0 | 99% | GU237484.3 |
| | CMS1 | 21948 | 21948 | 100% | 0.0 | 99% | CP022606.1 |
| | CMS3 | 21948 | 21948 | 100% | 0.0 | 99% | CP013934.1 |
| | CMU | 21948 | 21948 | 100% | 0.0 | 99% | CP013936.1 |
| | CMS2 | 21948 | 21948 | 100% | 0.0 | 99% | CP013726.1 |
| | CH2 | 19494 | 19494 | 99% | 0.0 | 96% | CP012873.1 |
| | 10M | 4684 | 4921 | 96% | 0.0 | 97% | KU363982.1 |
| | TN610 | 5160 | 5160 | 96% | 0.0 | 98% | HE818409.1 |
| *Lactobacillus fermentum* | Kg3 | 4903 | 4903 | 99% | 0.0 | 96% | AY697433.1 |

| | | | | | | | |
|---|---|---|---|---|---|---|---|
| Max. Score Maximum score; Score. Total: Total Score; GenBank access number at the GenBank database. | | | | | | | |

Having identified the strains of the prior art that comprise a nucleotide sequence encoding an enzyme able to produce EPS and presenting a high sequence homology with the nucleotide sequence (SEQ ID NO: 1) encoding the enzyme responsible for EPS synthesis (SEQ ID NO: 2) of the invention, Blast analysis was performed to check for the possible existence of bacterial strains that comprise plasmids containing the nucleotide sequence encoding the enzyme responsible for EPS synthesis of the invention. The results show the existence of strains of species *W. cibaria* that align with the complete plasmid sequence of the strain of the invention and that have a high identity with it (**Table 2**).

**Table 2. Homology of the plasmid of the invention compared to other plasmids present in other bacterial strains.**

| **Organism** | **Strain** | **Max. Score** | **Score total** | **Coverage (Query cover)** | **E-value** | **Identity** | **GenBank** |
|---|---|---|---|---|---|---|---|
| *Weissella cibaria* | CMS1 | 21948 | 21948 | 100% | 0.0 | 99% | CP022606.1 |
| | CMS3 | 21948 | 21948 | 100% | 0.0 | 99% | CP013934.1 |
| | CMU | 21948 | 21948 | 100% | 0.0 | 99% | CP013936.1 |
| | CMS2 | 21948 | 21948 | 100% | 0.0 | 99% | CP013726.1 |
| | CH2 | 19494 | 19494 | 99% | 0.0 | 96% | CP012873.1 |

| | | | | | | | |
|---|---|---|---|---|---|---|---|
| Max. Score Maximum score; Score. Total: Total Score; GenBank accession number at the GenBank database. | | | | | | | |

Analysed below is the presence of plasmids outside the genome in different strains of *Weissella* spp. whose genomes are completely or partially annotated in the databases analysed (NCBI) (**Table 3**). The result reveals that only 5 of them have plasmid annotations outside their genome.

**Table 3. Strains of Weissella spp. that contain plasmids outside their genome.**

| **Organism** | **Strain** | **Number of plasmids** |
|---|---|---|
| *W. cibaria* | CMS3 | 1 plasmid |
| *W. cibaria* | CMU | 2 plasmids |
| *W. cibaria* | CH2 | 6 plasmids |
| *W. koreensis* | KACC 15510 | 1 plasmid |
| *W. jogaejeotgali* | FOL01 | 1 plasmid |

As mentioned above, none of the strains known to date of the species *W. viridescens,* nor of the genus *Weisella,* have a plasmid outside the genome comprising the nucleotide sequence SEQ ID NO: 1 capable of encoding an enzyme (SEQ ID NO: 2) responsible for EPS synthesis, as described in the present invention.

### Example 3. Production and identification of EPS synthesized by strain CECT 9735.

The culture conditions for modulating the loss or non-loss of EPS production capacity by CECT 9735 strain of the invention are given below. For this purpose, serial cultivation was carried out in both liquid and solid medium for production and growth at different incubation temperatures: 30ºC and 37ºC, under anaerobic conditions.

The WVA production medium, both liquid and solid, comprise 10 g/L peptone, 8 g/L meat extract, 2 g/L K2HPO₄, 5 g/L NaC₂H₃O₂*3H₂O, 0.05 g/L MnSO₄*4H₂O, 0.2 g/L MgSO₄*7H₂O, 2 g/L (NH₄)3C₆H₇O₇, 1ml/L Tween 80, 0,04 g/L Red chlorophenol, 20 g/L Sacarose, 12 ml/L HCI 1N, pH = 6,5; and WVA growth medium, both liquid and solid, comprise 10 g/L peptone, 8 g/L meat extract, 2 g/L K₂HPO₄, 5 g/L NaC₂H₃O₂*3H₂O, 0,05 g/L MnSO₄*4H₂O, 0.2 g/L MgSO₄*7H₂O, 2 g/L (NH₄)3C₆H₇O₇, 1ml/L Tween 80, 0,04 g/L Red chlorophenol, 20 g/L Sacarose, 12 ml/L HCl 1N, pH = 7.2.

EPS production capacity after each serial cultivation was evaluated in a solid production medium.

The results show that no loss of EPS production capacity is observed after performing more than 20 serial cultivations under the above conditions, but the strain of the invention grows better when grown at a temperature of 30 ° C, with a higher production of EPS being observed in plates grown at 30°C than in plates grown at 37°C. Therefore, this is established as the optimal temperature for the evaluation of EPS synthesis by growing the strain of the invention.

The ability of pH to influence the loss of EPS production capacity by the strain of the invention was further tested. To do so, serial cultivations were carried out in the production medium, evaluating in parallel the growth in liquid or solid medium and at two different pH levels: 5 and 7.2. Incubation was performed at 30°C under anaerobic conditions.

The results demonstrate that the CECT 9735 strain of the invention loses its production capacity after five serial cultivations on solid WVA sucrose production medium when cultured at 30ºC in anaerobic conditions and at pH 5, whereas when grown at 30ºC in anaerobic conditions at pH de 7,2, it continues to synthesize EPS (**Fig. 1**). Therefore, the optimal conditions for EPS synthesis by CECT 9735 strain of the invention are growth in anaerobic conditions at a temperature of 30 ° C and at a pH of 7.2.

Then, we evaluated possible correlation between the loss of EPS production capacity and the presence of the nucleotide sequence (SEQ ID NO: 1) encoding an enzyme (SEQ ID NO: 2) capable of secreting EPS. To do so, the presence of the gene in the plasmid of the strain of the invention was identified by means of DNA amplification using PCR techniques. To avoid nonspecific amplifications, two pairs of primers were designed (**Table 4**) and DNA was amplified with the outermost pair of primers (SEQ ID NO: 4 and SEQ ID No: 5) in the coding sequence of the enzyme of the invention and, on the amplicon obtained, a second amplification was performed with the innermost pair of primers (SEQ ID No: 6 and SEQ ID No: 7) in the sequence of that gene.

**Table 4. Primers designed to amplify the nucleotide sequence (SEQ ID NO: 1) encoding the enzyme (SEQ ID NO: 2) responsible for synthesis of EPS of the invention.**

| **Name** | **Sequence** | **SEQ ID NO:** |
|---|---|---|
| **Direct 1** | CATTGATGACTCGCTTGGCG | 4 |
| **Reverse 1** | GCCATGAATTGCCCATCGTC | 5 |
| **Direct 2** | GCATTTTGGGGATATTACAG | 6 |
| **Reverse 2** | GTATAACGAATTGGAGCATC | 7 |

**Fig. 2** shows that the nucleotide sequence (SEQ ID NO: 1) of the CECT 9735 strain of the invention is detected only in the DNA plasmid (line 5 of Fig. 2), showing the nucleotide sequence (SEQ ID NO: 1) and not in the extraction of genomic DNA (line 4 of Fig. 2) extraction. As seen in Fig. 2, in the strain of the invention CECT 9735, cultivated under conditions in which it loses EPS production ability, SEQ ID NO: 1 is not detected in either genomic DNA (Line 2 of the Fig. 2) or in plasmid DNA (Fig Line 3. 2) extraction. This corroborates that the loss of the nucleotide sequence (SEQ ID NO: 1) is correlated with the loss of EPS production capacity.

Once the optimal culture conditions for the CECT 9735 strain of the invention were established, the next step was to identify the EPS secreted. This polymer has been characterized in terms of its elemental composition, bond types, molecular size and infrared spectrum.

The polymer produced by the CECT 9735 strain of the invention is mainly composed of a mixture of glucose and mannose. The approximate percentage of each of these saccharides is 70%, most preferably 73% for glucose and 20%, more preferably 24% for mannose.

**Table 5. Composition of the EPS secreted by strain CECT 9735 of the invention.**

| **Monosaccharides** | **%** | **Molar ratio** |
|---|---|---|
| Arabinose | 3.5 | 0.1 |
| Mannose | 24 | 1.0 |
| Glucose | 73.8 | 3.1 |
| Recovery | 101.3 | |

In accordance with this result, the types of bonds presented are glucose-mannose bonds deduced by the method of obtaining partially methylated alditol acetate (PMAA) (**Table 6**).

**Table 6. Types of bonds presented by the EPS synthesized by CECT 9735 strain of the invention.**

| | Ratio Ara:Man:Glu (0,1:1:3,1) | | |
|---|---|---|---|
| **Monosaccharide** | **PMAA** | **Types of bonds** | **Area (%)** |
| Glc or Man | 1,5-Ac₂-2,3,4,6-Me₄-Hex | Hex*π*-(1→ | 7.1 |
| Man | 1,2,5-Ac₃-3,4,6-Me₃-Man | →2)-Man*p*-(1→ | 1.3 |
| Man | 1,3,5-Ac₃-2,4,6-Me₃-Man | →3)-Man*p*-(1→ | 0.5 |
| Glc or Man | 1,5,6-Ac₃-2,3,4-Me₃-Hex | →6)-Hex*p*-(1→ | 81.7 |
| Glc or Man | 1,4,5,6-Ac₄-2,3-Me₂-Hex | →4.6)-Hex*p*-(1→ | 0.5 |
| Man | 1,3,5,6-Ac₄-2,4-Me₂-Man | →3.6)-Man*p*-(1→ | 9.1 |

Fourier-transform infrared spectroscopy (in cm-¹) of the EPS of the invention, showed a maximum absorbance peak at 1013.8 cm-¹, followed by a broad peak at 3298 cm⁻¹. Intense bands were also observed at approximately 2910, 2069, 1648.7, 1456, 1412, 1351, 1237 and 912 cm⁻¹.

In addition, we compared the EPS of the invention, with an EPS obtained from cultures of the strain *W. cibaria* CECT 7032. In the case of *W. cibaria* CECT 7032, the synthesized EPS contains less than 5% mannose and is composed primarily of glucose (**Table 7**).

**Table 7. Composition of EPS secreted by strain W. cibaria CECT 7032**

| **Monosaccharides** | **%** | **Molar ratio** |
|---|---|---|
| Mannose | 4.3 | 1.0 |
| Glucose | 65 | 15.1 |
| Recovery | 69.3 | |

The bonds presented by the EPS produced by strain *W. cibaria* CECT 7032 are mainly glucose, deduced using the partially methylated alditol acetate (PMAA) method (**Table 8**).

**Table 8. The bonds presented by the EPS produced by strain W. cibaria CECT 7032.**

| | Ratio Man:Glu (1:15,1) | | |
|---|---|---|---|
| **Monosaccharide** | **PMAA** | **Types of bonds** | **Area (%)** |
| Glc or Man | 1,5-Ac₂-2,3,4,6-Me₄-Hex | Hex*p*-(1 → | 6.4 |
| Man | 1,2,5-Ac₃-3,4,6-Me₃-Man | →2)-Man*p*-(1→ | 2.1 |
| Man | 1,3,5-Ac₃-2,4,6-Me₃-Man | →3)-Man*p*-(1→ | 0.9 |
| Glc or Man | 1,5,6-Ac₃-2,3,4-Me₃-Hex | →6)-Hex*p*-(1→ | 79.1 |
| Glc | 1,4,5,6-Ac₄-2,3-Me₂-Glc | 44,6)-Glc*p*-(1→ | 1.6 |
| Man | 1,2,5,6-Ac₄-3,4-Me₂-Man | →2,6)-Man*p*-(1→ | 1.5 |
| Glc | 1,3,5,6-Ac₄-2,4-Me₂-Man | →3,6)-Glc*p*-(1→ | 8.4 |

Regarding the molecular weight of the polymer, the EPS produced by the strain of the invention has a single peak, mean molecular weights of Mw =126.1 kDa and Mn=66.6 kDa and a polydispersion index of 1.9 (**Table 9**).

**Table 9. Molecular weight and polydispersion index of EPS obtained by CECT 9735 strain of the invention.**

| **Strain CECT 9735** | **Mw (kDa)** | **Mn (kDa)** | **Lp** |
|---|---|---|---|
| Replicate 1 | 117.6 | 58.3 | 2.02 |
| Replicate 2 | 127.6 | 66.8 | 1.91 |
| Replicate 3 | 133.0 | 74.6 | 1.78 |
| Medium | 126.1±7.8 | 66.6±8.2 | 1.9±0.12 |

In the case of *W. cibaria* CECT 7032, two peaks were obtained with different molecular weight sizes. Peak 1: Mw = 43753kDa and Mn = 30173 kDa and a polydispersion index of 1.45. Peak 2: Mw = 1057kDa and Mn = 700 kDa and a polydispersion index of 15.2 (**Table 10**).

**Table 10. Molecular weight and EPS polydispersion index obtained by strain W. cibaria CECT 7032.**

| | **Peak 1** | | | **Peak 2** | | |
|---|---|---|---|---|---|---|
| ***W. cibaria* CECT 7032** | Mw (kDa) | Mn (kDa) | Ip | Mw (kDa) | Mn (kDa) | Ip |
| **Replicate 1** | 40560 | 28060 | 1.44 | 996 | 662 | 1.5 |
| **Replicate 2** | 39340 | 26000 | 1.51 | 1031 | 688 | 1.5 |
| **Replicate 3** | 51360 | 36460 | 1.41 | 1144 | 749 | 1.53 |
| **Medium** | 43753±66 | 30173±55 | 1.45±0,05 | 1057±77 | 700±45 | 1.51±0,02 |

Both peaks have molecular weights greater than those of the EPS produced by CECT 9735 strain of the invention, which confers on the EPS secreted by that strain different physical characteristics to those of the EPS secreted by strain *W. cibaria* CECT 7032.

### Example 4. Production yields of EPS obtained by growing strain CECT 9735 of the invention compared to other known strains.

The production of EPS in different strains of the genus Weissella has been evaluated, specifically in 2 strains of the species *W. cibaria* and 2 strains of *W. viridescens,* comparing them to strain *W. viridescens* CECT 9735 of the invention.

For this purpose, starting with a preinoculum of each of the analysed strains, they are inoculated in VW growth medium and grown at pH 6.5, at 30°C for 16h in anaerobic conditions. From the preinoculum, they are inoculated on VW production medium and are grown at pH 7.2, under anaerobic conditions at 30°C.

After 48 h, the culture is centrifuged and the supernatant is recovered. Two volumes of EtOH are added and cold precipitated for 16h. Once the EPS is precipitated, it is recovered by centrifugation and freeze dried.

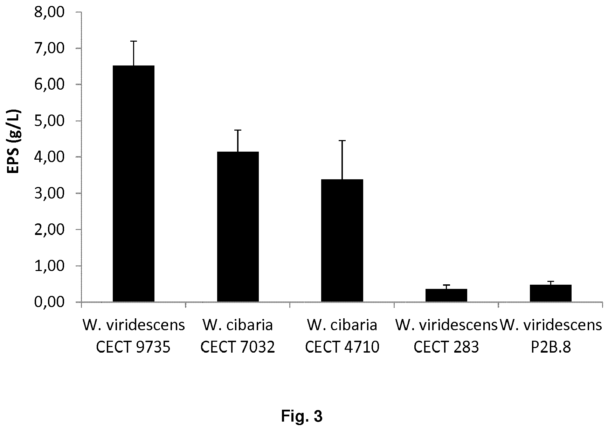

As seen in **Fig. 3** and **Table 11,** strain CECT 9735 of the invention is capable of producing 50% more EPS cultivated in the same conditions as the strains of *W*. *cibariaor* even strains of the same species, *W. viridescens,* which have low EPS production capacity. This result is totally unexpected, as it is the first description of EPS production capacity in the species *W. viridescens* and, furthermore, it is the first description of the presence of a nucleotide sequence encoding an enzyme capable of producing EPS in that bacterial species

**Table 11. EPS production by different strains of the genus Weissella.**

| | **Media (g/L)** | **SD** |
|---|---|---|
| ***W. viridescens* CECT 9735** | 6.52 | 0.68 |
| ***W. cibaria* CECT 7032** | 4.14 | 0.61 |
| ***W. cibaria* CECT 4710** | 3.39 | 1.06 |
| ***W. viridescens* CECT 283** | 0.37 | 0.11 |
| ***W. viridescens* P2B.8** | 0.49 | 0.09 |

## Claims

1. Bacterial strain comprising the SEQ ID NO: 1.

2. The bacterial strain according to claim 1 wherein the SEQ ID NO: 1 is comprised in an extrachromosomal plasmid, preferably the extrachromosomal plasmid comprises the SEQ ID NO: 3.

3. The bacterial strain according to any of the claims 1 and 2 wherein the SEQ ID NO: 1 encodes an enzyme capable of producing exopolysaccharides, wherein preferably, the enzyme comprises the SEQ ID NO: 2.

4. The bacterial strain according to any of the claims 1 to 5 **characterized by** belonging to the genus *Weissella,* preferably belonging to the species *Weissella viridescens,* more preferably is the strain *W. viridescens* CECT 9735.

5. Nucleotide sequence comprising the SEQ ID NO: 1.

6. Polypeptide sequence encoded by the nucleotide sequence according to claim 5, wherein preferably the polypeptide sequence comprising the SEQ ID NO: 2.

7. Vector comprising the nucleotide sequence according to claim 5, wherein the vector is preferably a plasmid.

8. Plasmid comprising the SEQ ID NO: 3.

9. Host cell comprising the nucleotide sequence according to claim 5, the polypeptide sequence according to claim 6, the vector according to claim 7, or the plasmid according to claim 8.

10. Use of the bacterial strain according to any of the claims 1 to 4, the nucleotide sequence according to claim 5, the polypeptide sequence according to claim 6, the vector according to claim 7, the plasmid according to claim 8, or the host cell according to claim 9, for exopolysaccharide production.

11. Method for producing exopolysaccharides comprising the following steps:
a) culture the bacterial strain according to any of the claims 1 to 4, or the host cell according to claim 9, in a culture medium; preferably at a pH in the range from 6.5 to 7.5, preferably 7.2; and at a temperature in the range from 28 ° C to 35 ° C, preferably 30 ° C; and
b) extraction of the exopolysaccharides synthesized by the cell in step a).

12. Exopolysaccharide obtainable by the method according to claim 11.

13. Exopolysaccharide **characterised by** comprising glucose, mannose and arabinose, in a molar ratio in the range from 3.1: 1: 0.1, preferably wherein the exopolysaccharide comprising at least the following percentages by weight: 30-45% C; 1-10%H; 1-15% N and 0.05-2% S; and an average molecular weight in the range from 100 to 150 kDa, an average molecular mass by weight in the range from 50 to 100 kDa and a polydispersity index in the range from 1.5 and 2.

14. Use of the exopolysaccharide obtainable by the method according to claim 11, or the exopolysaccharide according to claim 13, in the food, pharmaceutical, cosmetic and biomedical industries, preferably in the food industry as thickener, stabilizer, emulsifier, gelling agent, hydrocolloid, and/or inclusion bodies.

15. Composition comprising the exopolysaccharide obtainable by the method according to claim 11, or the exopolysaccharide according to claim 13, and active substances, excipients and/or additives.


SEQUENCE LISTING

<110>  Sant Dalmai, S.A.U.

<120>  Weissella viridescens strain and uses thereof for the synthesis of
       exopolysaccharides

<130>  EP3113.15

<160>  7

<170>  PatentIn version 3.5

<210>  1
<211>  3042
<212>  DNA
<213>  Weissella viridescens

<400>  1
ttgcaagacg agtcacagaa gtttagaaaa aagatgtaca agtccggaaa gttttgggta      60

gctgccggtg ctttgtttgt tgggctggca ttcgctggta acacgcaggc tgatactgta     120

ttaccaagtg aacaacgtgc aacggagacg acacagacca gtgaagacac gtccgccact     180

aagacgccgg catcggcgtc gacttcaagc tcagtcaatg ttgacacgag tgacctgcct     240

gacagttcaa gtacggcagt tgatagtaca agtgcaagtg caagcgtagt gagtgatagc     300

gtcgctgtgc cagatactgg atcacaattt acgagttcgt cagcgtcaat gtcatcatca     360

tttgttaagt catcactagc ggcaacaact agtgatgctt ctggcagtca gtcggcggcg     420

gtgactagcg caaccgttag ttcggtggcc acgagtagtt cagcatcttc agtgacaaca     480

gccacaagcg aatcagcagt gataagcagc gccgtgtcag atggttacca tgatgaaggt     540

ggtgattggg tctattatcg agctggaaaa aagttagtcg gtcgacaaac gattgatacg     600

tttgcggttt actttgacgc cgatggcaaa caagtcaagg gtgattggcg tgaaagtgat     660

ggtaaacgtg cgtattatga tggacaagaa ggacgagcat taacgcaaac gcaagcagtc     720

aatggcgtta tctacggttt taatcaaagc ggctatcaaa tcaagaatga tttcggccaa     780

acagcgaatc gagatacgta ttatttcgac gcacaaggtc atgttgtcac gggaatacaa     840

acaattgcaa acaaggttta tgattttgat gagcaaggtc gaatgctgaa aggcattgcc     900

acgtcagttg atgacaagat gatgtatttt gatgatcaaa caggtgttgg acaaccggct     960

gatcatcctg aattcaaccc tgaaacggaa ccggttcctg acgacaatat caaacataat    1020

gcagcacatg gtacgacacc agcagatttt gattcgatgg ctggctacct gacggctgat    1080

acttggtatc gcccaaccga tattttggaa aatggtgaga cgtggcgcga atcgcaacca    1140

actgaatttc gaccactgtt agcaacttgg tggccaacaa aacaaactca ggccgattac    1200

gtgaactaca tgaatcacgc attagatatg tcaaatgcaa gtgtgtcaga tgccgattca    1260

gaagccacgc taactgcggc aaccgatgct attcaagcgg ccgttgagca ccaaattacg    1320

gtgcgccaat caacggcctg gttacgtgaa ttaatggccg cgtttgttgt gacacagcca 1380

cagtggaata aaaccagtga agatgttaat gatgatcatt tgcaaggtgg ggcgctaaca 1440

tttgagaata acggcgacac agacgctaat tcggattatc gcctcatgaa tcgcacgcca 1500

acaaatcaga ctggtgaacg cttgtatcac attgatgact cgcttggcgg ttacgaatta 1560

ttgctggcaa atgacgttga caattcaaat ccacaagttc aggcagaaca attgaattgg 1620

ttgtactact tgatgcattt tggggatatt acagctgatg atccggatgc aaattttgat 1680

gccatacgga ttgatgcggt cgataatgtc gatgctgatt tacttcaact agcagctcag 1740

tatttccgtg atgcctatgg catggccacg actgacgcga catcaaataa gcatctttca 1800

atacttgagg attggagcca taacgatccg gcgtatatgc aagcacacgg caatgatcaa 1860

ttaacgatgg atgattatat gcacacacag ctgatttggt cattaaccaa gccagaggca 1920

caacgtggca ccatggcacg ctttatggac ttctatctca ccaaccgtgc taatgatgat 1980

acagaaaaca cggcgcaacc tagttattcg tttgtgcgtg cccatgatag cgaagtgcaa 2040

acagtcattg ctgaaatcgt gacgaagcta catccagaag caggaaacgg gttaatgcct 2100

acggaagaac aaatggcaga agcgtttaag atttacaatg cggaccaaaa gaaggccgtt 2160

aaaacttaca cgcactacaa tatgccatct gcatacgcca tgctgttaac gaacaaggat 2220

gttattccac gaatttacta tggtgacttg tacactgacg atgggcaatt catggcgaca 2280

aaatcaccct attttgatgc gatttcggct atgttacaag cgcgcacgaa gtatgtagct 2340

ggtggacaaa cgatggcggt tgaccagcac gacgtcttga ctagcgttcg gtttggtaag 2400

ggtgccatga cggccagtga tttaggaaat gctgagactc ggactgaggg tgtgggatta 2460

attattagca acaacccaaa gttgcaattg ggacaacaag ataacgtggt gttacacatg 2520

ggacttgcgc acgcgaatca agcattccga gcagttgtac taacgaccgc gaccggatta 2580

accatttata atgacgatga tgctccaatt cgttataccg ataatagggg tgatttaatt 2640

ttcaataacc atgacgtata tggcgtgttg aatccacaag tgtcaggctt cttggcaatg 2700

tgggtgccaa ctggtgcacc agcgaaccag gatgcgcgat ctactgcgtc aaccaacagt 2760

tcaacggatg gatctgccta ccattctaat gcggctttag atagtcaagt catctttgaa 2820

tcattttcga atttccaggc tatgccaaca agtcatgaca cgtacaccaa cgttgtgtta 2880

gccaatcatg ctgaccagtt acacgattgg ggaataactt cggtacagtt agcgccacaa 2940

taccggtctt caaccgacgg aacctttttg gatgcgatta ttcaaaatgg ctatgccttc 3000

actgaccgtt atgatttagg gtttggtacg ccaactaagt at 3042

<210> 2
<211> 1014
<212> PRT
<213> Weisella viridescens

<400> 2

Leu Gln Asp Glu Ser Gln Lys Phe Arg Lys Lys Met Tyr Lys Ser Gly
1               5                   10                  15

Lys Phe Trp Val Ala Ala Gly Ala Leu Phe Val Gly Leu Ala Phe Ala
            20                  25                  30

Gly Asn Thr Gln Ala Asp Thr Val Leu Pro Ser Glu Gln Arg Ala Thr
        35                  40                  45

Glu Thr Thr Gln Thr Ser Glu Asp Thr Ser Ala Thr Lys Thr Pro Ala
    50                  55                  60

Ser Ala Ser Thr Ser Ser Ser Val Asn Val Asp Thr Ser Asp Leu Pro
65                  70                  75                  80

Asp Ser Ser Ser Thr Ala Val Asp Ser Thr Ser Ala Ser Ala Ser Val
                85                  90                  95

Val Ser Asp Ser Val Ala Val Pro Asp Thr Gly Ser Gln Phe Thr Ser
            100                 105                 110

Ser Ser Ala Ser Met Ser Ser Ser Phe Val Lys Ser Ser Leu Ala Ala
        115                 120                 125

Thr Thr Ser Asp Ala Ser Gly Ser Gln Ser Ala Ala Val Thr Ser Ala
    130                 135                 140

Thr Val Ser Ser Val Ala Thr Ser Ser Ser Ala Ser Ser Val Thr Thr
145                 150                 155                 160

Ala Thr Ser Glu Ser Ala Val Ile Ser Ser Ala Val Ser Asp Gly Tyr
                165                 170                 175

His Asp Glu Gly Gly Asp Trp Val Tyr Tyr Arg Ala Gly Lys Lys Leu
            180                 185                 190

Val Gly Arg Gln Thr Ile Asp Thr Phe Ala Val Tyr Phe Asp Ala Asp
        195                 200                 205

Gly Lys Gln Val Lys Gly Asp Trp Arg Glu Ser Asp Gly Lys Arg Ala
    210                 215                 220

Tyr Tyr Asp Gly Gln Glu Gly Arg Ala Leu Thr Gln Thr Gln Ala Val
225                 230                 235                 240

Asn Gly Val Ile Tyr Gly Phe Asn Gln Ser Gly Tyr Gln Ile Lys Asn
                245                 250                 255

Asp Phe Gly Gln Thr Ala Asn Arg Asp Thr Tyr Tyr Phe Asp Ala Gln
            260                 265                 270

Gly His Val Val Thr Gly Ile Gln Thr Ile Ala Asn Lys Val Tyr Asp
        275                 280                 285

Phe Asp Glu Gln Gly Arg Met Leu Lys Gly Ile Ala Thr Ser Val Asp
    290                 295                 300

Asp Lys Met Met Tyr Phe Asp Asp Gln Thr Gly Val Gly Gln Pro Ala
305                 310                 315                 320

Asp His Pro Glu Phe Asn Pro Glu Thr Glu Pro Val Pro Asp Asp Asn
                325                 330                 335

Ile Lys His Asn Ala Ala His Gly Thr Thr Pro Ala Asp Phe Asp Ser
            340                 345                 350

Met Ala Gly Tyr Leu Thr Ala Asp Thr Trp Tyr Arg Pro Thr Asp Ile
        355                 360                 365

Leu Glu Asn Gly Glu Thr Trp Arg Glu Ser Gln Pro Thr Glu Phe Arg
    370                 375                 380

Pro Leu Leu Ala Thr Trp Trp Pro Thr Lys Gln Thr Gln Ala Asp Tyr
385                 390                 395                 400

Val Asn Tyr Met Asn His Ala Leu Asp Met Ser Asn Ala Ser Val Ser
                405                 410                 415

Asp Ala Asp Ser Glu Ala Thr Leu Thr Ala Ala Thr Asp Ala Ile Gln
            420                 425                 430

Ala Ala Val Glu His Gln Ile Thr Val Arg Gln Ser Thr Ala Trp Leu
        435                 440                 445

Arg Glu Leu Met Ala Ala Phe Val Val Thr Gln Pro Gln Trp Asn Lys
    450                 455                 460

Thr Ser Glu Asp Val Asn Asp Asp His Leu Gln Gly Gly Ala Leu Thr
465                 470                 475                 480

Phe Glu Asn Asn Gly Asp Thr Asp Ala Asn Ser Asp Tyr Arg Leu Met
                485                 490                 495

Asn Arg Thr Pro Thr Asn Gln Thr Gly Glu Arg Leu Tyr His Ile Asp
        500                 505                 510

Asp Ser Leu Gly Gly Tyr Glu Leu Leu Leu Ala Asn Asp Val Asp Asn
    515                 520                 525

Ser Asn Pro Gln Val Gln Ala Glu Gln Leu Asn Trp Leu Tyr Tyr Leu
    530                 535                 540

Met His Phe Gly Asp Ile Thr Ala Asp Asp Pro Asp Ala Asn Phe Asp
545                 550                 555                 560

Ala Ile Arg Ile Asp Ala Val Asp Asn Val Asp Ala Asp Leu Leu Gln
                565                 570                 575

Leu Ala Ala Gln Tyr Phe Arg Asp Ala Tyr Gly Met Ala Thr Thr Asp
            580                 585                 590

Ala Thr Ser Asn Lys His Leu Ser Ile Leu Glu Asp Trp Ser His Asn
        595                 600                 605

Asp Pro Ala Tyr Met Gln Ala His Gly Asn Asp Gln Leu Thr Met Asp
    610                 615                 620

Asp Tyr Met His Thr Gln Leu Ile Trp Ser Leu Thr Lys Pro Glu Ala
625                 630                 635                 640

Gln Arg Gly Thr Met Ala Arg Phe Met Asp Phe Tyr Leu Thr Asn Arg
                645                 650                 655

Ala Asn Asp Asp Thr Glu Asn Thr Ala Gln Pro Ser Tyr Ser Phe Val
            660                 665                 670

Arg Ala His Asp Ser Glu Val Gln Thr Val Ile Ala Glu Ile Val Thr
        675                 680                 685

Lys Leu His Pro Glu Ala Gly Asn Gly Leu Met Pro Thr Glu Glu Gln
    690                 695                 700

Met Ala Glu Ala Phe Lys Ile Tyr Asn Ala Asp Gln Lys Lys Ala Val
705                 710                 715                 720

Lys Thr Tyr Thr His Tyr Asn Met Pro Ser Ala Tyr Ala Met Leu Leu
                725                 730                 735

Thr Asn Lys Asp Val Ile Pro Arg Ile Tyr Tyr Gly Asp Leu Tyr Thr
            740                 745                 750

Asp Asp Gly Gln Phe Met Ala Thr Lys Ser Pro Tyr Phe Asp Ala Ile
        755                 760                 765

Ser Ala Met Leu Gln Ala Arg Thr Lys Tyr Val Ala Gly Gly Gln Thr
    770                 775                 780

Met Ala Val Asp Gln His Asp Val Leu Thr Ser Val Arg Phe Gly Lys
785                 790                 795                 800

Gly Ala Met Thr Ala Ser Asp Leu Gly Asn Ala Glu Thr Arg Thr Glu
                805                 810                 815

Gly Val Gly Leu Ile Ile Ser Asn Asn Pro Lys Leu Gln Leu Gly Gln
            820                 825                 830

Gln Asp Asn Val Val Leu His Met Gly Leu Ala His Ala Asn Gln Ala
        835                 840                 845

Phe Arg Ala Val Val Leu Thr Thr Ala Thr Gly Leu Thr Ile Tyr Asn
    850                 855                 860

Asp Asp Asp Ala Pro Ile Arg Tyr Thr Asp Asn Arg Gly Asp Leu Ile
865                 870                 875                 880

Phe Asn Asn His Asp Val Tyr Gly Val Leu Asn Pro Gln Val Ser Gly
                885                 890                 895

Phe Leu Ala Met Trp Val Pro Thr Gly Ala Pro Ala Asn Gln Asp Ala
            900                 905                 910

Arg Ser Thr Ala Ser Thr Asn Ser Ser Thr Asp Gly Ser Ala Tyr His
        915                 920                 925

Ser Asn Ala Ala Leu Asp Ser Gln Val Ile Phe Glu Ser Phe Ser Asn
    930                 935                 940

Phe Gln Ala Met Pro Thr Ser His Asp Thr Tyr Thr Asn Val Val Leu
945                 950                 955                 960

Ala Asn His Ala Asp Gln Leu His Asp Trp Gly Ile Thr Ser Val Gln
                965                 970                 975

Leu Ala Pro Gln Tyr Arg Ser Ser Thr Asp Gly Thr Phe Leu Asp Ala
            980                 985                 990

Ile Ile Gln Asn Gly Tyr Ala Phe Thr Asp Arg Tyr Asp Leu Gly Phe

995                1000                1005

Gly Thr  Pro Thr Lys Tyr
    1010

<210>  3
<211>  12120
<212>  DNA
<213>  Weissella viridescens

<400>  3
atacttagtt ggcgtaccaa accctaaatc ataacggtca gtgaaggcat agccattttg        60

aataatcgca tccaaaaagg ttccgtcggt tgaagaccgg tattgtggcg ctaactgtac       120

cgaagttatt ccccaatcgt gtaactggtc agcatgattg gctaacacaa cgttggtgta       180

cgtgtcatga cttgttggca tagcctggaa attcgaaaat gattcaaaga tgacttgact       240

atctaaagcc gcattagaat ggtaggcaga tccatccgtt gaactgttgg ttgacgcagt       300

agatcgcgca tcctggttcg ctggtgcacc agttggcacc cacattgcca agaagcctga       360

cacttgtgga ttcaacacgc catatacgtc atggttattg aaaattaaat cacccctatt       420

atcggtataa cgaattggag catcatcgtc attataaatg gttaatccgg tcgcggtcgt       480

tagtacaact gctcggaatg cttgattcgc gtgcgcaagt cccatgtgta acaccacgtt       540

atcttgttgt cccaattgca actttgggtt gttgctaata attaatccca caccctcagt       600

ccgagtctca gcatttccta aatcactggc cgtcatggca cccttaccaa accgaacgct       660

agtcaagacg tcgtgctggt caaccgccat cgtttgtcca ccagctacat acttcgtgcg       720

cgcttgtaac atagccgaaa tcgcatcaaa atagggtgat tttgtcgcca tgaattgccc       780

atcgtcagtg tacaagtcac catagtaaat tcgtggaata acatccttgt tcgttaacag       840

catggcgtat gcagatggca tattgtagtg cgtgtaagtt ttaacggcct tcttttggtc       900

cgcattgtaa atcttaaacg cttctgccat ttgttcttcc gtaggcatta acccgtttcc       960

tgcttctgga tgtagcttcg tcacgatttc agcaatgact gtttgcactt cgctatcatg      1020

ggcacgcaca aacgaataac taggttgcgc cgtgttttct gtatcatcat tagcacggtt      1080

ggtgagatag aagtccataa agcgtgccat ggtgccacgt tgtgcctctg gcttggttaa      1140

tgaccaaatc agctgtgtgt gcatataatc atccatcgtt aattgatcat tgccgtgtgc      1200

ttgcatatac gccggatcgt tatggctcca atcctcaagt attgaaagat gcttatttga      1260

tgtcgcgtca gtcgtggcca tgccataggc atcacggaaa tactgagctg ctagttgaag      1320

taaatcagca tcgacattat cgaccgcatc aatccgtatg gcatcaaaat ttgcatccgg      1380

atcatcagct gtaatatccc caaaatgcat caagtagtac aaccaattca attgttctgc      1440

ctgaacttgt ggatttgaat tgtcaacgtc atttgccagc aataattcgt aaccgccaag      1500

cgagtcatca atgtgataca agcgttcacc agtctgattt gttggcgtgc gattcatgag 1560
gcgataatcc gaattagcgt ctgtgtcgcc gttattctca aatgttagcg ccccaccttg 1620
caaatgatca tcattaacat cttcactggt tttattccac tgtggctgtg tcacaacaaa 1680
cgcggccatt aattcacgta accaggccgt tgattggcgc accgtaattt ggtgctcaac 1740
ggccgcttga atagcatcgg ttgccgcagt tagcgtggct tctgaatcgg catctgacac 1800
acttgcattt gacatatcta atgcgtgatt catgtagttc acgtaatcgg cctgagtttg 1860
ttttgttggc caccaagttg ctaacagtgg tcgaaattca gttggttgcg attcgcgcca 1920
cgtctcacca ttttccaaaa tatcggttgg gcgataccaa gtatcagccg tcaggtagcc 1980
agccatcgaa tcaaaatctg ctggtgtcgt accatgtgct gcattatgtt tgatattgtc 2040
gtcaggaacc ggttccgttt cagggttgaa ttcaggatga tcagccggtt gtccaacacc 2100
tgtttgatca tcaaaataca tcatcttgtc atcaactgac gtggcaatgc ctttcagcat 2160
tcgaccttgc tcatcaaaat cataaacctt gtttgcaatt gtttgtattc ccgtgacaac 2220
atgaccttgt gcgtcgaaat aatacgtatc tcgattcgct gtttggccga aatcattctt 2280
gatttgatag ccgctttgat taaaaccgta gataacgcca ttgactgctt gcgtttgcgt 2340
taatgctcgt ccttcttgtc catcataata cgcacgttta ccatcacttt cacgccaatc 2400
acccttgact tgtttgccat cggcgtcaaa gtaaaccgca aacgtatcaa tcgtttgtcg 2460
accgactaac ttttttccag ctcgataata gacccaatca ccaccttcat catggtaacc 2520
atctgacacg gcgctgctta tcactgctga ttcgcttgtg gctgttgtca ctgaagatgc 2580
tgaactactc gtggccaccg aactaacggt tgcgctagtc accgccgccg actgactgcc 2640
agaagcatca ctagttgttg ccgctagtga tgacttaaca aatgatgatg acattgacgc 2700
tgacgaactc gtaaattgtg atccagtatc tggcacagcg acgctatcac tcactacgct 2760
tgcacttgca cttgtactat caactgccgt acttgaactg tcaggcaggt cactcgtgtc 2820
aacattgact gagcttgaag tcgacgccga tgccggcgtc ttagtggcgg acgtgtcttc 2880
actggtctgt gtcgtctccg ttgcacgttg ttcacttggt aatacagtat cagcctgcgt 2940
gttaccagcg aatgccagcc caacaaacaa agcaccggca gctacccaaa actttccgga 3000
cttgtacatc ttttttctaa acttctgtga ctcgtcttgc aaaattatat ccccgtattc 3060
tattcatatc cccaaggcct tggtgtaggg ttcaccttaa cgcggacata atctctctgt 3120
caattttttc acaatgtcat attagaaccg gttccatttt attcatatgc aagcataatg 3180
agactcgtta gttaaattag tgctttttat ccaaattacc ttaagtcaca gtaccccagc 3240
cttactcaga acattagttc cacgggtacc ctctagttta caactgtaaa tctaatatac 3300
atgaaatata aaaaacatac aaaaattaat tattgatatt tcacaagcca ccgttattgg 3360
tagcacaagt aaatacaaaa aagccgatga attaacagct actaactgtc aattcatcga 3420

ccataaagat attatgttaa ttaaaatgtt gcgcatgtct gttgctaata aacttaattg 3480

cgatcatacc gacaaccatc atcatcccaa gcaaaaagac tgtccattcg cccaattcag 3540

acaccccata tccaaaggca attgttataa tcggcgcaaa taaatctgtc gcaaagaaag 3600

aaataccaaa tacccgtccc aagtatgctg gcgcactctc ttgttgacgg accgtattaa 3660

tcttaataac aaacgatgca cgagcaaacc cattcaagat aaccagaccg ataaataccc 3720

atatttcttg aataaatgtg ccgatgataa aagccccacc tagaacaaac agatcccaat 3780

aaagcgtatt taacgaattg aattttggta agcgcgctaa aattatgccc gcaataatac 3840

ctgcaactgc cgccaaagtt aaggccgtag aatatgacga agctgagcca tggtaattat 3900

gctgaatcag aaatggcatc gcaataataa atccttcaaa gaagaaattg ctccacatcg 3960

ttagtaacat cgattcaaat aattttggtt gggacaacac gtatttgtat ccaccaacta 4020

aactggctag aaatgactcc gatggcatat taggatcttt tggttgcggc tgatatctaa 4080

tcgccaagtt caacaagacc gtaacgacaa gcatcacacc aaacaactgc aaaaacgttt 4140

gcaagttaat ccattttaag tgaagcaaga gaccaccaac taatggtcca aatgcttgtc 4200

ccgtcgagaa agctgcgcta gcccatgcgt taaaacgggc caaggcactt gacttaatga 4260

catctggcaa aattgcgcgc gcggctgggt acgcaaacga gacatccaca ttcaaaatga 4320

aagttagtaa aaataatacc cagagctgtg gcttagtcgg atcaatcagc aacgataatg 4380

ttaaagcgcc gatggcacca aataaatctg cgcctaacaa cacccattta cgattaaagc 4440

gatccagcac tgaaccaaca taaacatcat ttaataagta aaccaaaaag ccgaaacctg 4500

ttagccaacc taaaattttt gcattgccat agctagacac taaaaaccaa ttcaggccaa 4560

accgatagac accatctcca aacaccgacg caaaattcga cgcaaggagt ggaaaactaa 4620

atttcgctaa gcgcttatca ttttcaatca tatcagttca cctcctacct ataattctct 4680

ccagtgtatc atagctcaca catcagtaac accgaaaaaa agaccatgta cgcagtatta 4740

ttaccgcaca catggtcttt ttatcgtatt aattaaagaa acttgataaa gacaacacca 4800

acagccatca caacaacacc caaaatttga ataccgttaa cactagcctt ggctgaacgc 4860

caccagccaa attgttgtac aagcattgaa ccaacaattt gaccgatcaa tcccatcgta 4920

atcgtcaaac cagcaccaat ctttggcact gacaaaacac tggcgaagac gaatagacca 4980

cccaagatac cgccaagcca gttccacgac ttagcttgct ttaactcaga caccttaggc 5040

aagcgcttat caaccaccaa agcaacgatg gtaatcaaga taaacccaac aaagaatgaa 5100

atgaatgagc cttgtgctgg ggcttgcaat aacgctccca agtgaccgtt aattgcttgt 5160

tgcattgctg acaacgttcc agtaaaaatt gcccagattt gccaaccaag caagactggc 5220

tttgtttcac tctcctgaat ggccttatcc attgcttcca catccttgtt taggacgttg 5280

ggcaaaacaa ccgctacaac aacaccagca atcaacacaa ttagcccgat gactcgcatt 5340

aatgtcaaag gtgtttgtgc cgtaccaaac caaccaaagg tatcaataat ggtacccatt 5400

aaaatttgac ccaaaattgg caaaataacc gtctgaattg cacccaagcg tgggaatagc 5460

aacacgttcg atgtcaaata gacagcacca agtacaccac ctagccaaat ccaagccgga 5520

tttgaactga tgaacgctcc tgatgggaac acacggtgtg acataatcgc gctcaaaacg 5580

cctaaaaaga ttgaaccaat caaaaatgaa attgccgctg aaatgaatgg tgaaccaacc 5640

accttcttca tgtcagcgtt aattggattt tggtttgcca acataaaacc agcaaagagt 5700

gacaaaccta gaaatacaag aaccacaatt cttctcctcg aaacttactt ataattatta 5760

ttgctttatt attgtagttc tttgaaaatg aaatgcaagc attttgagca agcgagtttg 5820

tgaaatttgg ttctttttca atttcaatct cttatatcga tagggtttct agcaaccgtt 5880

cactcataaa gtcttcacaa gcttgtgaaa agaatctccg ttaattgttt cagcgaatca 5940

aaaaaatcgc cattcaaacc aaattgaata gcgatcgtct aaacaccata cgtcaggtgt 6000

tacgtttaat tatcttttcg tttcaaatct tcccgtgtgc caccacgacg ctttcgggca 6060

ggcagtaagt atcgcccaag taagaatacg gctaggaaca caataattgg tcccacggcc 6120

aatttccacc aattacgtgg ttgttgccaa tcattgcccg cgactgccga tccagttaac 6180

tcagctacct gtgcggcagt gactgttaac gttttagtgg ctgtcttagt catatcagct 6240

gttttaacct gaaccttaac ggtatactta cctggcttaa tcgacttcac tttgtcttta 6300

atttcaaagt taaaagttga ctttggtgcc atgtcaacgc gatgtgatgt cttttcaaaa 6360

actgttttac cgttattgtc ctgaaccttg gttgccacag ttgcatcatt cacgagctga 6420

ttagcgttat ttgttaactt aacaccaatg acgcgtgcgc tcgtcaattg tgtcagcgca 6480

gcacggccga ttttcacatc tgccgatacc ggcgttgtat cattttgcaa aataacaggt 6540

atctcagagg cagtgtttgc actcgtcgtc cggacgaatg acaccccacc aactaactga 6600

ccttgaatcg catggtccgg cattttaatc gtcccagcaa ctgacgtttc accagcagcc 6660

ggtacagtca ccgtattttg aatggtaata taatcaccaa ctttatacgg cacttgacct 6720

gtcacgtttt ggctacccgt cacataattg atgatgccat tatcacgggt agtggctgtc 6780

ccgcctgtta actgcaagtc aatcggcttt tttgtgttat ttgtaatata gaatgtcaca 6840

tgttcttctt gacctggtgc caactttaga tcaatcgctg ataagttatt cttattaatc 6900

tgagtagctg gaatcgaagc cgttatatca taatccggca gtttagtgtc atccgcatga 6960

cccgtcacac caaccgcacc aatcgtaatt gttgatgcga tagcaagtat tgctgttcgc 7020

aagttcataa acccgtaccc ctaattttct ctaactaaag attaattata gaatgtctgc 7080

taataaaatg cacgcagtag ccatggcaaa taccattaat ttgttagtca ttaactagcc 7140

attcggttgg cttttggaaa tcacccaata actaatagcc gcaatcgcac caatgcttat 7200

caaaccaatt ccgactaccc acaccgaaaa gccccgtact tgctgctcct ggtgcgccat 7260

cctgaccgtt accggttgat tccaggactg ggtcagccct gatgttttca catgaacggc 7320

tagacgatac tttcccggcg gtaattgttt atttttacgg gagactgtta gtggccacga 7380

tgtatttggt gctaacgaaa tttgtttgaa cgttgccgtc atgatcggtt tattagttgc 7440

aatatcagtt agtgacgccg tacctgagag atcatttaca aatcgtccaa ctttattatt 7500

aatctgaaac gcaatgcctg tccgattcgg taacgaaatc gccgttggct gcgacaacga 7560

caacgccact ttaggtagtt gcgcctgcgc acgggcatga acagcaatcg tatatgacag 7620

ggcgtgcgct aactgaattt tgtcattgat atcctgtttc gctttgaccg ctgtaaatga 7680

caccccgcct gccatcaagc caatgtccgc tgtattaggc attgtaagcg ttgctgtgac 7740

cgttttttgg ccgtctgcgg ggatgacaac ggtctttgcg atttgtaggt aatttgtcat 7800

ctgaatcgtc ggcgctcgat tctccgtcaa tggttgatat gtaacacgac cgctacttga 7860

tgtagtggca ttactcggat taaccgcaat ccgcagatcg tcatctgtat ggttagttag 7920

aataaaagtt aactcttttg tagtacctgg tgctaataac aaatcatagt atgccgtatt 7980

agacggttgc cgttgttcag aaatagccgt atcgattgca actgaaaaat cacgttcggc 8040

agcttgaata ctgccaccaa acaaaaggat gctccccaaa agcattaata aaaatcgtcg 8100

catttccctc tcctgatgcg atgcccgcaa aaaattgccc ggctcggtat gtttaactgt 8160

gaaaaaataa gtattctgcc gttactaaaa aaccgaggca ttcgccccgg cccgttagtt 8220

ttaattttaa tccaagaaat cacgcaattg ctttgaacgt gatgggtggc gtagcttacg 8280

aagtgccttg gcttcaattt gacgaatacg ctcacgcgta accccaaata ccttaccaac 8340

ttcttccaac gtacgtgtac gaccatcttc caaaccaaaa cgcaaacgca aaacgttctc 8400

ttcacgatcc gtcaacgtat ccagcactga ttccaattgt tccttcaaca tttgataagc 8460

ggctgaatct gcaggcgaaa tggcttcatt atcttcaata aagtcaccca aatgtgagtc 8520

atcctcttca ccaattggtg tttccaatga cacaggctct tgggcaatct tcaagatatc 8580

acgaaccttg tctgttacga tgtccatctc ggcaccgatt tcttctggcg ttggctcacg 8640

tcccaagtct tgtagcaagt tacgttgaat acgaatcaac ttgttaatcg tttcaaccat 8700

gtgcacagga atacgaattg tacgagcttg gtcggcaatt gcacgtgtaa tcgcttgacg 8760

aatccaccat gtggcatacg ttgaaaactt aaatccctta cggtaatcaa acttttcaac 8820

cgccttcatc aatcccatgt ttccttcttg aatcaagtcc aagaattgca taccacgacc 8880

aacataacgc ttagcaattg acaccaccaa acgcaaatta gcttctgcca attcttgctt 8940

ggctgtgtca tcgccttgct caatacgctc ggccaaagca acttcttcgt caccagtcag 9000

caatgagaca cgaccaattt ccttcaagta catccggaca gggtcattaa tcttcatacc 9060

agttggcgca tcagcagctt gcttcaactc cttttctgat aacttagttt tcttgttttc 9120

caatgcacgc gcagttggct ctcccttttc gtcaacgatt gagattcctg cggattcgat 9180

cttttcgtat aattcatcga tttgatcagc attcaaacgc attggcttaa ccaaagcatc 9240

ttggagggca ttttccttga tttctttatc agctttgaac tccttgatga tgttcttcac 9300

tgccttatca aactcaactt gatcaaactt agctgagata ggtgcttgct tttctgccat 9360

agcggcctcc ttaagttact tgtgtgtttg ttgttcacga agcgcagcca aatattcaac 9420

acttagctga gcaactaatg catcattgtg catttgatta gcttctttaa tttgtcgttg 9480

taacctatta attctatcag ataatggtgc gtcatgacta ataaattgaa tatattcatc 9540

aacttgcgaa cgatccgtga ataattcagg cgcgtaagta tccaacgtgg ccaatattct 9600

agtcaaatcc gctttctgta cgtagtccat aaagcccgcc atatcggtga ttggctggtt 9660

accatgctct gatcggtaac ctgatgcgag catcataatc gtttcatacg gcacatcagc 9720

aaaagtgaaa tctggtgtgt tggttacttg tagccagaca tcattatcct gcaacatcca 9780

agctagcaac attcgctcag cttgttcaac ccgcgaaatt gtcggtgcaa ccggtgctgg 9840

gacaggcgca ggcgcactat cttgccactc atcatatggt gttgcaccaa aatcagcatc 9900

tgcaacgcgc tcactttggc gtggacgctt gttattcgaa cgtcgcggat tagaccgccg 9960

agcttgttgt tgcaataaca acggccgtaa ctgatcctgt aaggcatcca aaccgatgtt 10020

aaactcctca ccaatttggc gcaaataggt ttcacgcata atcggctcag tgacagttgc 10080

aatcgtccct aacacatcac taacataggc aaacatttct gtttgatttg ttaaatcacg 10140

acccatccgt aaataacgga tattaaaagc aatcggatct tccgtattat gtgatagcac 10200

attcaaaaat gctgacacat cattcgcacg taaaaattca tctggatctt ggttatctgg 10260

aatatgcaca accgtggctg tcattttacc gttctgtgcc actaagtcga gtgctcgttt 10320

tgtcgccctt tgcccagcgt cgtcagcatc ataactgata ctaatcttgt cggccattcg 10380

tgacaaaacc tggacttgtt ccgttgtcaa agatgttccc attgaggcaa caccattttg 10440

aacacctgct tggaatgcag caatgacatc cataaatcct tcgaataaga caacttgttt 10500

ttgcttacgg atggcgcctt tggccaaatc caaattaaac aattcttgcg acttattaaa 10560

taacggactc tctggcgaat tcatatactt cgcctgtgaa tcgtcattcg ataaacgacg 10620

tccagaaaag gcgattgggt gccccgcagc atcacgaatt gtaaatagta cacgattcgt 10680

gaaacggtca tgcaatgacc catcgtccca ctcaataaat agttcagacg ctcgcaataa 10740

ttgatagtcc acgccctgtt cattgaaata attgtataaa acatcatttt caggggcata 10800

ccctaacata aaggcatcaa tcgtgccgtc atctaaacca cgctcgtgta agtacgtcag 10860

tgcagtatca ccagcgtcgg tgttaacgag taaatgatgg tatagcttcg ttgcatctgc 10920

atacaatgat cgtaaggctt ttgtttgtgg atcaactgtt tcagttggct catctgccgt 10980

gtaggatgca tcaaggtcga cgcccgcaaa cggtgcaacc ttcgcaactg cttgtgggaa 11040

cgttgcatta tcaatatcca tgataaagct aaacacgttg cccccacgac cgcatgagaa 11100

gcagtgataa atttgcttat cttcattaac tgaaaacgag ggcgtacgtt catcgtggaa 11160

cgggcacagc ccaaacagat tacgcccttg ttttttcagt tgtacatatg gcgaaatgac 11220

atcaacaatg ttgacacgct cgcgaatttc ttcgacaagg tgttctggaa tacgtgttgc 11280

gatagtcatc acccccatct cttaaaatga acttttccag ctcttattat tatacgccgg 11340

aacgtacaat atgcaagtag cttgtcaagc ccctataatc tagtaaaagg ccggtatatt 11400

aacattttgt atttcagctt aaaatattat gtaaactaac gcaatctata tttcaagtaa 11460

attatctgcc attttggggt tgttccattt gacaaatacc actagtaaaa accgcattgt 11520

ttggcactaa tcgaatcgac cagtcacgca aacaatgcgg ttcatttttc aaagcaatga 11580

aatgtctaac gactattcaa gattactgtg acgtgctttc ataaacgact ctgtaatttg 11640

atagcgttgc atataatcca tcgtaaagat gtcaccaaac aaccaaaccg actgttcaaa 11700

taaactgccc atcggttgtt tcgtagctgt atcggcatca gcaaacttgc ttttgcctgc 11760

cagcagtgtc acaaattgtg actggcgtgc aattgtacta tccgtaccgg tgctccatag 11820

ccagattgtc gcacctacgt cactagcagt tgccgcaaat tgaacaagtt gcgttgtttc 11880

accagaagaa gacgcaatga ctaacaaatc agacgctgta attgccggtg tcgttgtttc 11940

acccacaaca aacgcttgtt tacccaactg cgtaagacgc attgcaaacg actttagcgc 12000

cagacctgat cgtccagcac cagccaggaa aatgcgttct gattgattta cgagaccgag 12060

tagcgcttcg tcattcacat catctacatt ttgtgccaac tccgttaaaa ctgtttgcca 12120

<210> 4
<211> 20
<212> DNA
<213> Artificial Sequence

<220>
<223> Forward primer 1

<400> 4
cattgatgac tcgcttggcg 20

<210> 5
<211> 20
<212> DNA
<213> Artificial Sequence

<220>
<223> Reverse primer 1

<400> 5
gccatgaatt gcccatcgtc 20

<210>  6
<211>  20
<212>  DNA
<213>  Artificial Sequence

<220>
<223>  Forward primer 2

<400>  6
gcattttggg gatattacag                                                    20


<210>  7
<211>  20
<212>  DNA
<213>  Artificial Sequence

<220>
<223>  Reverse primer 2

<400>  7
gtataacgaa ttggagcatc                                                    20